(19) Europäisches Patentamt
European Patent Office
Office européen des brevets

(11) EP 2 785 697 B1

(12) **EUROPÄISCHE PATENTSCHRIFT**

(45) Veröffentlichungstag und Bekanntmachung des Hinweises auf die Patenterteilung:
**03.02.2016 Patentblatt 2016/05**

(21) Anmeldenummer: **12795740.5**

(22) Anmeldetag: **28.11.2012**

(51) Int Cl.:
***C07D 231/12*** (2006.01) ***C07D 231/16*** (2006.01)
***C05G 3/08*** (2006.01)

(86) Internationale Anmeldenummer:
**PCT/EP2012/004918**

(87) Internationale Veröffentlichungsnummer:
**WO 2013/079197 (06.06.2013 Gazette 2013/23)**

(54) **N-(1H-PYRAZOLYL-METHYL)FORMAMIDE, VERFAHREN ZU IHRER HERSTELLUNG UND IHRE VERWENDUNG ALS NITRIFIKATIONSINHIBITOREN**

N-(1H-PYRAZOLYL-METHYL) FORMAMIDES, PROCESSES FOR THEIR PREPARATION AND THEIR USE AS NITRIFICATION INHIBITORS

N-(1H-PYRAZOLYL-MÉTHYL) FORMAMIDES, LEURS PROCÉDÉS DE PRÉPARATION ET LEUR UTILISATION EN TANT QU' INHIBITEURS DE NITRIFICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.12.2011 DE 102011120098**

(43) Veröffentlichungstag der Anmeldung:
**08.10.2014 Patentblatt 2014/41**

(73) Patentinhaber: **SKW Stickstoffwerke Piesteritz GmbH**
**06886 Lutherstadt Wittenberg (DE)**

(72) Erfinder:
- **RADICS, Ute**
  **06901 Kemberg (DE)**
- **NICLAS, Hans-Joachim**
  **12435 Berlin (DE)**
- **BENDER, Christoph**
  **10249 Berlin (DE)**

(74) Vertreter: **Schneider, Michael**
**Heisse Kursawe Eversheds**
**Brienner Strasse 12**
**80333 München (DE)**

(56) Entgegenhaltungen:
**WO-A1-2005/035509**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 785 697 B1

Printed by Jouve, 75001 PARIS (FR)

## Beschreibung

Die vorliegende Erfindung betrifft N-(1H-Pyrazolyl-methyl)formamide, Verfahren zu ihrer Herstellung, Düngemittelzusammensetzungen, die sie aufweisen, sowie ihre Verwendung zur Hemmung bzw. Steuerung der Nitrifikation.

Die Anwendung von Stickstoffdüngemitteln, die Nitrifikationsinhibitoren (im Folgenden auch als Nitrifikationshemmer bezeichnet) enthalten, ist dadurch besonders vorteilhaft, weil die im allgemeinen rasch ablaufende Nitrifikation, d.h. die mikrobielle Umwandlung von Amid- und Ammoniumstickstoff zu Nitratstickstoff, durch die Gegenwart derartig wirkender Substanzen auf diese Weise verzögert wird. Durch die so gesteuerte Nitratbildung im Boden werden eine zu intensive Nitratbildung und damit die Akkumulation von Nitrat vermieden. Eine so bewirkte Zurückdrängung des sonst entstehenden Nitratüberschusses im Boden konserviert den Düngerstickstoff in Form von Ammoniumionen, sorgt damit für eine höhere Düngereffizienz und führt gleichzeitig zu einer erheblich verringerten Umweltbelastung im Rahmen einer Stickstoffdüngung.

So ist bekannt, dass Nitrat im Gegensatz zum Ammonium im Boden nur äußerst schlecht sorbiert wird und demzufolge der Auswaschung bzw. der Verlagerung in tiefere Bodenschichten unterliegt. Andererseits führt die infolge einer Nitratanreicherung im Boden verstärkt einsetzende Denitrifikation zu gasförmigen Stickstoffverlusten, wobei neben molekularem Stickstoff auch solche Oxide wie Stickstoffmonoxid bzw. Lachgas emittiert werden, die zur Versauerung des Bodens beitragen bzw. als klimaverändernde Gase bekannt sind.

Durch die Hemmung bzw. Steuerung der Nitrifikation wird gleichzeitig einer Nitratanreicherung im Grundwasser entgegengewirkt.

Gerade in der Reduzierung der beschriebenen Verlustpotentiale liegt der wirtschaftliche Vorteil von mit Nitrifikationsinhibitoren versehenen Stickstoffdüngemitteln, der sich u.a. in der erhöhten Nährstoffeffizienz dieser Dünger niederschlägt. Um das zu unterstreichen sei erwähnt, dass N-Verluste von konventionellen Stickstoffdüngern durchaus unter ungünstigen Bedingungen Größenordnungen von bis zu 40% und mehr annehmen können.

## Stand der Technik

Als wirksame Nitrifikationshemmer wurden unter anderem stickstoffhaltige heterocyclische Verbindungen, bevorzugt aus der Reihe der 1H-Pyrazole und der 1,2,4-Triazole, vorgeschlagen, z.B. in US 3,635,690, US 4,969,946, US 4,523,940, EP-A 166 420, JP-OS 71-04135, US 3,697,244.

Ein wesentlicher Nachteil der meistens sehr gut nitrifikationshemmend wirkenden Pyrazolderivate besteht in ihrer relativ hohen Flüchtigkeit bzw. besonders bei am N-1-Stickstoffatom substituierten Verbindungen in ihrer Hydrolyseempfindlichkeit, in deren Folge zwar noch wirksame, aber wieder flüchtige Derivate gebildet werden. Aus diesen Gründen lässt sich eine Anwendung bei festen Düngemitteln, insbesondere in Kombinationen mit Harnstoff nicht ohne weiteres realisieren. Zur Beseitigung dieses Mangels sind Vorschläge zur chemischen Abwandlung der 1-N-unsubstituierten Basispyrazole bekannt (DE-OS 27 45 833, EP-A 508 191, DD 292 232, DE-OS 40 18 395, EP-A 160 804, DE-OS 37 04 359, SU 1 470 737, DE-OS 44 46 194, EP-A 808 297). Mit dem Rückgang der Flüchtigkeit durch Derivatisierung ist jedoch in der Regel zugleich auch ein Abfall in der Wirkung als Nitrifikationsinhibitor verbunden. Außerdem sind solche Herangehensweisen mit einem Mehraufwand an Syntheseschritten gekoppelt, die fast durchgängig zu einer Kostenbelastung führten, so dass ihre Anwendung ökonomisch nicht mehr sinnvoll ist.

Andere Lösungen zur Reduzierung der Flüchtigkeit von nitrifikationshemmend wirkenden Pyrazolen bestehen in der Ausnutzung des eigentlich bekannten Prinzips der Salzbildung mit Säuren (EP-A 529 473, WO 98/05 607, DE-OS 4018 395). Sowohl die mineralsauren Salze der nitrifiziden Basispyrazole als auch nitrifizid wirkende Pyrazole, die am 1-N-Stickstoff hydrophile Gruppen tragen, können als Nitrifikationsinhibitoren noch nicht voll befriedigen, da sie eine zu geringe Hydrolysestabilität besitzen, wodurch die Wirkungsdauer im Boden und die Lagerstabilität herabgesetzt ist. Dies betrifft z.B. N-Glyoxylpyrazole (DE-A 37 04 359) oder N-Hydroxymethylpyrazole (SU-A 1 470 737). Mit der Einführung von verschiedenartigen lipophilen Gruppen in 1-N-Position (DE-OS 101 17 821 A1, DE-OS 101 35 423 A1) konnte zwar die Hydrolyseanfälligkeit dieser Pyrazolderivate beseitigt werden, jedoch sind diese Nitrifikationsinhibitoren bei niedrigen Konzentrationen nicht mehr ausreichend lang leistungsfähig. Ein weiterer Mangel besteht in den aufwendigen Herstellungsverfahren für diese Wirkstoffe.

In der DE 103 43 277 A1 werden auch Pyrazolderivate zur Steuerung der Nitrifikation beschrieben, es hat sich aber gezeigt, dass ihre Löslichkeit in Wasser oder wäßrigen Lösungen, ein Konditionierungsmittel enthaltend, nicht ausreicht, um eine sinnvolle technische Anwendbarkeit in Bezug auf die Aufbringung auf Düngergranulate oder ähnliche Formen zu realisieren.

## Aufgabenstellung

Der vorliegenden Erfindung lag die Aufgabe zugrunde, Wirkstoffe zur Hemmung bzw. Steuerung der Nitrifikation von Ammoniumstickstoff zu entwickeln, die sich auf Grund der gezeigten Nachteile durch eine Kombination von hoher

Wirksamkeit in Bezug auf die Hemmung bzw. Steuerung der Nitrifikation und sehr guter Wasserlöslichkeit auszeichnen.

Die Wirkstoffe sollen weiterhin aufgrund ihrer geringen Flüchtigkeit und ihrer Hydrolysestabilität alle Applikationsverfahren, aber auch alle Möglichkeiten zur Einarbeitung in oder Aufbringung auf mineralische stickstoffhaltige Dünger zulassen und außerdem eine ausgezeichnete Residualwirkung aufweisen und unabhängig davon auch eine Anwendung zu organischen Düngern zulassen. Des Weiteren stellt sich der Bedarf nach Verbindungen, die eine Aufbringung auf Düngemittel in Form von Granulaten oder ähnlichen Formen durch eben die erwähnte Kombination aus hoher Wirksamkeit und sehr guter Wasserlöslichkeit technisch sinnvoll erscheinen lassen. Gleichzeitig sollten diese Wirkstoffe durch eine unkomplizierte, ökonomisch sinnvolle Synthese herstellbar sein.

## Beschreibung der Erfindung

Diese Aufgabe wurde erfindungsgemäß durch die in den Ansprüchen definierten N-(1H-Pyrazolyl-methyl)-formamide entsprechend Formel I gelöst.

Es hat sich überraschenderweise gezeigt, dass die erfindungsgemäßen N-(1H-Pyrazolyl-methyl)-formamide entsprechend Formel I die geforderte Kombination aus hoher Wirksamkeit als Wirkstoffe zur Hemmung bzw. Steuerung der Nitrifikation von Ammoniumstickstoff und sehr guter Wasserlöslichkeit aufweisen, was nicht zu erwarten war.

Wie aus Tabelle 1 ersichtlich wird, tritt nur bei den erfindungsgemäßen N-(1H-Pyrazolylmethyl)-formamiden diese Kombination der benötigten Anforderungen auf. Prinzipiell war davon auszugehen, dass das einzusetzende Säureamid (siehe hierzu die Verfahrensbeschreibungen) einen organischen Rest (siehe hierzu DE 103 43 277 A1), möglichst einen Aromaten, aufzuweisen hat, um eine gewisse Wirkung als Wirkstoffe zur Hemmung bzw. Steuerung der Nitrifikation von Ammoniumstickstoff zu erreichen. Gleichzeitig stellt sich aber dann das Problem der sehr geringen Wasserlöslichkeit. Wird der o.g. organische Rest durch eine polare Aminogruppe ersetzt, steigt erwartungsgemäß auch die Wasserlöslichkeit, die Wirkung als Wirkstoffe zur Hemmung bzw. Steuerung der Nitrifikation von Ammoniumstickstoff fällt aber rapide ab.

Daher war es nicht zu erwarten, dass die erfindungsgemäßen N-(1H-Pyrazolyl-methyl)-formamide entsprechend Formel I die gewünschte Kombination aus hoher Wirksamkeit als Wirkstoffe zur Hemmung bzw. Steuerung der Nitrifikation von Ammoniumstickstoff und sehr guter Wasserlöslichkeit aufweisen.

**Tabelle 1: Vergleich von Wirksamkeit und Wasserlöslichkeit**

| Substanz | Wirksamkeit (K-Wert bei 0,2 ppm) | Wasserlöslichkeit [g/kg] |
|---|---|---|
| 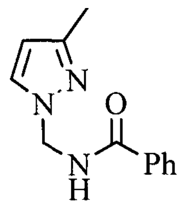 | 0,8 | < 0,1 |
| 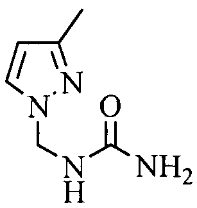 | 0 | 209 |
| 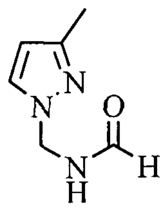 | 0,76 | 3188 |

Es hat sich zusätzlich gezeigt, dass diese Verbindungen neben der sehr guten nitrifikationshemmenden Wirkung den außerordentlichen Vorteil einer sehr geringen Hydrolyseneigung besitzen, wodurch Wirkstoffverluste bei der Oberflächenapplikation, aber auch bei der Lagerung von Dünger-Wirkstoff-Gemischen nahezu ausgeschlossen sind. Im Gegensatz zu bereits bekannten Azolderivaten liegt der besondere Vorteil der erfindungsgemäßen Verbindungen darin,

dass sie nicht nur problemlos mit vielen nitrathaltigen N-Düngemitteln wie Ammonsulfatsalpeter und sauer reagierenden Düngern wie beispielsweise Ammoniumsulfat kombiniert werden können, ohne dass sie vorher in ihre Salze überführt werden müssen, um Wirkstoffverluste infolge Flüchtigkeit zu verhindern, sondern aufgrund der beschriebenen Eigenschaften besonders verlustfrei mit hamstoffbasierten Düngemitteln formuliert werden können. Dies ist umso bemerkenswerter als bekannt ist, dass Harnstoff stets von geringen Anteilen an freiem Ammoniak begleitet ist, der die sonst schwächer basischen Azolderivate aus ihrem für die Wirkung unerlässlichen Verbund mit Harnstoff verdrängt und somit zu unterkritischen Konzentrationen an Nitrifikationsinhibitor führt, die eine ausreichende Nitrifikationshemmung nicht gewährleistet.

Überraschenderweise kommt die nitrifizide Wirkung der erfindungsgemäßen N-(1H-Pyrazolyl-methyl) formamide auch bei sehr geringen Wirkstoffkonzentrationen zur vollen Entfaltung, was nicht vorhersehbar war. So müssen beispielsweise schwerflüchtige Alkyl-1H-1-pyrazolylmethanamin-Derivate (DE 198 22 340 A1) in der Regel mit der doppelten Aufwandmenge eingesetzt werden, um die gleiche nitrifikationshemmende Wirkung wie die erfindungsgemäßen N-(1 H-Pyrazolyl-methyl)-formamide zu erzielen.

Die erfindungsgemäßen N-(1H-Pyrazolyl-methyl)-formamide entsprechen gemäß Anspruch 1 der allgemeinen Formel (I)

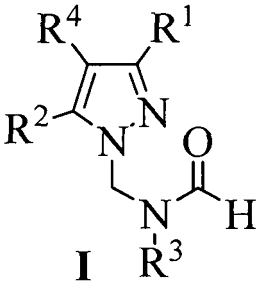

I

wobei $R^4$ = H, Halogen, $C_1$-$C_4$-Alkyl oder $C_3$-$C_8$-Cycloalkyl ist,

$R^1$ und $R^2$ unabhängig voneinander für H, $C_1$-$C_4$-Alkyl oder $C_3$-$C_8$-Cycloalkyl steht

$R^3$ für $C_1$-$C_4$-Alkyl, $C_3$-$C_8$-Cycloalkyl oder $C_6$-$C_{10}$-Aryl oder H, steht.

Die Erfindung betrifft ferner Verfahren zur Herstellung der erfindungsgemäßen N-(1H-Pyrazolyl-methyl)-formamide, wie in den Ansprüchen **4** bis **11** definiert, die in Anspruch **12** definierte Verwendung der N-(1 H-Pyrazolyl-methyl)-formamide sowie Zusammensetzungen, die diese N-(1H-Pyrazolyl-methyl)-formamide enthalten, wie in Anspruch **13** bis **17** definiert.

Weitere vorteilhafte und/oder bevorzugte Ausführungsformen der Erfindung sind Gegenstand der Unteransprüche.

Vorzugsweise stellt $R^1$ einen Methylrest und $R^4$ = $CH_3$, oder $R^1$ Methyl und $R^4$ = H oder Cl dar, und außerdem ist unabhängig davon bei einer weiteren Ausführungsform $R^2$ vorzugsweise H.

Der Begriff »Alkyl« bezieht sich hier, und zwar in jeder Kombination mit beliebigen anderen Gruppen, insbesondere (sofern nicht anders definiert) auf eine Alkyl-Gruppe, die 1 bis 4 Kohlenstoffatome aufweist, z.B. eine Methyl-, Ethyl-, n-Propyl-, Isopropyl-, n-Butyl-, Isobutyl-, oder tert-Butyl-Gruppe. Die Alkyl-Gruppen mit der entsprechenden Kohlenstoffanzahl können geradkettig oder verzweigt sein.

Der Begriff »Aryl« bezieht sich auf eine aromatische cyclische Gruppe, die einen oder mehrere Ringe aufweist, und durch ein Gerüst gebildet wird, das 6 bis 10 Kohlenstoffatome enthält. Selbstverständlich können im Falle von mehreren Ringen einer der Ringe oder auch mehrere Ringe ganz oder teilweise hydriert sein (ein Beispiel dafür ist die 1,2,3,4,-Tetrahydronaphthalen-1-yl-Gruppe).

Der Begriff »Cycloalkyl« bezieht sich auf eine gesättigte cyclische Gruppe, die einen oder mehrere Ringe aufweist, die ein Gerüst bilden, welches 3 bis 8 Kohlenstoffatome enthält, z.B. eine Cyclopropyl-, Cyclobutyl-, Cyclopentyl-, Cyclohexyl-Gruppe.

Der Begriff »Halogen« umfasst stets Fluor, Chlor, Brom und lod.

Ein weiterer Gegenstand der vorliegenden Erfindung ist die Verwendung der erfindungsgemäßen N-(1 H-Pyrazolyl-methyl)-formamide zur Hemmung bzw. Regelung der Nitrifikation, wobei die erfindungsgemäß vorgeschlagenen Verbindungen in bekannter Weise als Nitrifikationsinhibitoren mit festen ammonium- und/oder amidhaltigen Düngemitteln kombinierbar sind. Als Stickstoffdüngemittel werden vorzugsweise Ammoniumsalze, wie z.B. Kalkammonsalpeter, Ammonsulfatsalpeter, Ammoniumnitrat, Ammoniumsulfat oder Harnstoff eingesetzt. Möglich ist auch der Einsatz

der erfindungsgemäßen N-(1H-Pyrazolyl-methyl)amide in Kombination mit organischen und flüssigen Düngemitteln. Aufwandmengen von 0,01 bis 10,0 Gew.%, z.B. 0,01 bis 5,0 Gewichtsprozent, insbesondere 0,01 bis 3,0 Gewichtsprozent, berechnet auf den reduzierten Stickstoffgehalt des Düngers, d. h. auf Ammonium- und Carbamid-Stickstoff, genügen für den praktischen Einsatz. Selbstverständlich können die erfindungsgemäßen N-(1H-Pyrazolyl-methyl)-formamide gegebenenfalls mit weiteren üblichen Nitrifikationsinhibitoren und/ oder Ureaseinhibitoren in Kombination verwendet werden. Wirkstoffformulierungen, Lösungen und Konzentrate, welche die erfindungsgemäßen N-(1H-Pyrazolyl-methyl)-formamide aufweisen, sind ein weiterer Gegenstand der Erfindung.

Die Art und Weise, wie die erfindungsgemäß vorgeschlagenen Nitrifikationsinhibitoren mit dem Düngemittel kombiniert werden, unterliegt keinen besonderen Beschränkungen. Es eignen sich sämtliche im Stand der Technik bekannten Verfahren. So können die N-(1H-Pyrazolyl-methyl)formamide bevorzugt allein oder im Gemisch mit anderen Zusatzstoffen als Lösung oder gemeinsam mit einem geeigneten Konditionierungsmittel, beispielsweise mit einem hydrophobierend wirksamen paraffinischen Konditionierungsmittel, auf die Oberfläche der Düngemittelgranulate aufgebracht werden, wobei das Lösemittel (vorzugsweise aprotische Lösemittel, wie z.B. Ester, Öle, Ketone, Ether etc.) z.B. durch Trocknung entfernt wird.

Es hat sich außerdem überaschenderweise gezeigt, dass die erfindungsgemäßen N-(1 H-Pyrazolyl-methyl)-formamide eine hervorragende Löslichkeit in Wasser oder wässrigen Lösungen, ein Konditionierungsmittel enthaltend, aufweisen. Im Vergleich zu den Pyrazolderivaten nach DE 103 43 277 A1 ist die Löslichkeit im Durchschnitt mehr als doppelt so hoch.

Alternativ hierzu können die erfindungsgemäßen N-(1 H-Pyrazolyl-methyl)-formamide allein oder im Gemisch mit anderen Zusatzstoffen zur homogenen Verteilung im Dünger in die Düngemittelschmelze oder -slurry während des Formgebungsprozesses, z.B. zur Herstellung von Granulat, eingebracht werden. Flüssigen und organischen Düngern werden die erfindungsgemäßen Verbindungen vorzugsweise in Form einer geeigneten Formulierung zugegeben.

Es ist klar, das zur Formulierung übliche und dem Fachmann bekannte Hilfs-, Träger- und Streckstoffe oder eine Kombination dieser Mittel, oder auch oberflächenaktive Mittel, z.B. ein Benetzungs-, Dispersions-, Emulgierungs- oder Suspensionsmittel, verwendet werden können. Die jeweiligen Formulierungsverfahren entsprechen dem Stand der Technik und sind dem Fachmann bekannt.

Die erfindungsgemäßen Verbindungen zeichnen sich durch ausgezeichnete nitrifizide Eigenschaften auch bei geringen Aufwandmengen aus. Die Einführung des Restes -C(=O)-H gegenüber substituierten Pyrazolen, die in DE 103 43 277 A1 beansprucht werden, führt zu einem erheblichen Anstieg der Wasserlöslichkeit unter gleichzeitigem Erhalt der Hydrolysestabilität der Verbindungen und erfolgt ohne Einbußen ihrer nitrifiziden Wirkung. Aufgrund dieser Eigenschaften sind die erfindungsgemäßen N-(1H-Pyrazolyl-methyl)-formamide bevorzugt für eine Oberflächenapplikation auf den Düngemittelgranulaten geeignet.

Die erfindungsgemäß vorgeschlagenen N-(1H-Pyrazolyl-methyl)-formamide (I) (wobei $R^1$, $R^2$, $R^3$ sowie $R^4$ die oben genannte Bedeutung besitzen) sind durch eine einfache Dreikomponentenreaktion gemäß der allgemeinen Gleichung (1) zugänglich. Dazu werden die Reaktionspartner Azol II, Formaldehyd III (z.B. als Paraformaldehyd) und das Amid IV (wobei $R^1$, $R^2$, $R^3$ sowie $R^4$ die oben genannte Bedeutung besitzen) z.B. im Molverhältnis 1:3:3 bis 1:1:1 innig miteinander gemischt und z.B. 15 bis 500 Minuten auf z.B. 50 bis 200°C, vorzugsweise auf 80 bis 145°C, z.B. auch ohne Lösungsmittel in der Schmelze, erhitzt.

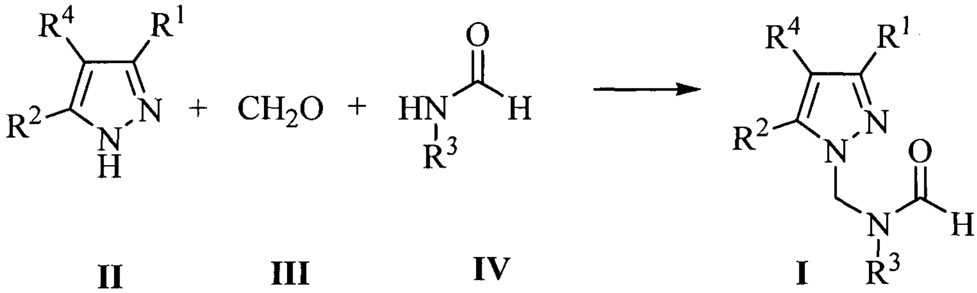

(1)

II III IV I

Eine weitere erfindungsgemäße Herstellungsmethode besteht in der bereits erwähnten Dreikomponenten Reaktion in einem Lösungsmittel. Dazu werden die Reaktionspartner Azol II, Formaldehyd III (z.B. als Paraformaldehyd) und das Amid IV (wobei $R^1$, $R^2$, $R^3$ sowie $R^4$ die oben genannte Bedeutung besitzen) z.B. im Molverhältnis 1:3:3 bis 1:1:1 innig miteinander gemischt und z.B. 15 bis 500 Minuten auf z.B. 50 bis 200°C, vorzugsweise auf 80 bis 145°C in einem Lösungsmittel erhitzt. Als Lösungsmittel kommen hierbei, ohne dass die Erfindung durch die Nennung oder Nichtnennung eingeschränkt wird, in Frage:

- Lösungsmittel aus der Gruppe der Alkane wie n-Pentan, n-Hexan oder n-Heptan u.a.

- Lösungsmittel aus der Gruppe der halogenierten Alkane oder Alkene wie Dichlormethan, Chloroform, Trichlorethan oder Trichlorethen u.a.
- Lösungsmitte aus der Gruppe der Säurederivate wie Ethylacetat, Ethylformiat, Dimethylformamid u.a.
- Lösungsmittel aus der Gruppe der Ether wie Diethylether, Tetrahydrofuran oder 1,4-Dioxan u.a.
- Lösungsmittel aus der Gruppe der Aromaten wie Benzol, Toluol oder Xylol u.a.

Die Umsetzung gemäß der allgemeinen Gleichung (1) kann auch in wässrigen Systemen, gegebenenfalls unter Zusatz von Lösungsvermittlern wie Alkoholen, beispielsweise Methanol oder Ethanol oder auch Acetonitril, unter Einsatz von wässriger Formaldehydlösung durchgeführt werden. Beispielsweise kann man die Reaktion bei Temperaturen von z.B. 40 bis 110°C in beispielsweise Wasser als Lösungsmittel und mit einem Molverhältnis von Azol II zu Formaldehyd und Amid IV von z.B. 1:3:3 bis 1:1:1 durchführen.

N-(1H-Pyrazolyl-methyl)-formamide (I) (wobei $R^1$, $R^2$, $R^3$ sowie $R^4$ die oben genannte Bedeutung besitzen) können auch erhalten werden, indem man die entsprechenden Hydroxymethyl-pyrazole (V) (wobei $R^1$, $R^2$ sowie $R^4$ die oben genannte Bedeutung besitzen) mit Amiden (IV) (wobei $R^3$ die oben genannte Bedeutung besitzt) gemäß der allgemeinen Gleichung (2) umsetzt, wobei das entsprechende Hydroxymethyl-pyrazol (V) mit dem Amid (IV) z.B. im Molverhältnis 1:1,0 bis 2,0, gegebenenfalls unter Zusatz von z.B. 0,2 bis 10% einer geeigneten Säure, vorzugsweise eines saueren Ionenaustauschers, in einem organischen Lösungsmittel, ausgewählt aus der Gruppe aromatische Kohlenwasserstoffe (wie z.B. Toluol), der Gruppe der Halogenkohlenwasserstoffe (wie z.B. Methylenchlorid) oder der Gruppe der Perfluorkohlenwasserstoffe (wie z.B. Perfluoroctan (PF5080)) oder ohne Lösungsmittel, bei Temperaturen von z.B. 40 bis 160°C unter Wasserabscheidung zur Reaktion gebracht wird.

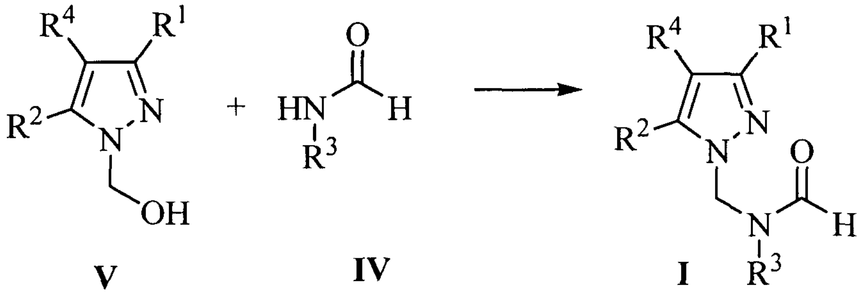

(2)

Die Hydroxymethyl-pyrazole (V) bzw. deren Herstellung sind aus der Literatur bekannt (J. Hüttel Chem. Ber. 85 (1952) 820, OS 28 35 158, OS 199 13 475, L. Maier Phosphorus Sulfur 33 (1987) 41).

Die Reaktionsprodukte fallen als kristalline Substanzen an. Bei der Synthese der erfindungsgemäßen Verbindungen entsprechend den Gleichungen (1) und (2) wurde das Auftreten von Isomeren in Bezug auf die Stellung des $R_1$- und $R_2$-Substituenten außer Acht gelassen, es zeigten sich jedoch gelegentlich durch etwas breitere Schmelzbereiche. Falls gewünscht, können die jeweiligen Isomeren mittels der üblichen, dem Fachmann bekannten Verfahren getrennt werden.

**Ausführungsbeispiel**

Die nachfolgenden Beispiele sollen die Erfindung ohne Beschränkung näher veranschaulichen.

**Darstellung von N-(1H-Pyrazolyl-methyl)-formamiden (I)**

Variante A: Das entsprechende 3-Methylpyrazol (0,1 mol), Formamid (5,18 g; 0,115 mol) und Paraformaldehyd (3,9 g; 0,13 mol) werden in einem Zweihalskolben mit Innenthermometer und Destillationsaufsatz vorgelegt und erwärmt. Die Reaktionsmischung wird solange bei 140 °C Innentemperatur gehalten, bis kein Wasser mehr abdestilliert (ca. 5 - 7 Stunden). Nach Abkühlen wird die Mischung säulenchromatographisch gereinigt. Man erhält die Produkte als farblose Öle, die mit der Zeit bei Raumtemperatur zu weißen Feststoffen erstarren.

Variante B: Das entsprechende 3-Methylpyrazol (0,1 mol), Formamid (5,18 g; 0,115 mol) und Paraformaldehyd (3,9 g; 0,13 mol) werden in einem Zweihalskolben mit Innenthermometer und Wasserabscheider vorgelegt. Die Mischung wird mit 50 ml Toluol versetzt und erwärmt. Die Reaktionsmischung wird solange in der Siedehitze gehalten, bis kein Wasser mehr abdestilliert (ca. 5 - 7 Stunden). Nach Abkühlen und Abdestillieren des Lösungsmittels wird die Mischung

säulenchromatographisch gereinigt. Man erhält die Produkte als farblose Öle, die mit der Zeit bei Raumtemperatur zu weißen Feststoffen erstarren.

Variante C: Das entsprechende N-Hydroxymethyl-3-methylpyrazol (0,1 mol) und Formamid (5,18 g; 0,115 mol) werden in einem Zweihalskolben mit Innenthermometer und Wasserabscheider vorgelegt. Die Mischung wird mit 50 ml Toluol versetzt und erwärmt. Die Reaktionsmischung wird solange in der Siedehitze gehalten, bis kein Wasser mehr abdestilliert (ca. 5 - 7 Stunden). Nach Abkühlen und Abdestillieren des Lösungsmittels wird die Mischung säulenchromatographisch gereinigt. Man erhält die Produkte als farblose Öle, die mit der Zeit bei Raumtemperatur zu weißen Feststoffen erstarren.

**N-((3(5)-Methyl-1H-pyrazol-1-yl)methyl)-formamid[1] 1**

$R_F$: 0,125 (Hexan Ethylacetat 1:3)
$^1$H NMR (500 MHz, *$CDCl_3$*) δ ppm 8.22 & 8.20 (s, 1H CH=O); 7.95 (s, 1H, NH); 7.54 (d, *J* = 1.8 Hz) & 7.39 (s, 1H, $CH_{ar}$-N); 6.03 (d, *J* = 1,8 Hz) & 6.00 (s, 1H, $CH_{ar}$); 5.47 & 5.43 (d, *J* = 6,6 Hz, 2H, $CH_2$); 2.43 & 2.24 (s, 3H, $CH_3$)
$^{13}$C NMR (125 MHz, *$CDCl_3$*) δ ppm 161.7 &161.1 (C=O); 149.3 & 139.4 $C_{q,ar}$; 131.6 $CH_{ar}$; 105.7 & 105.6 $CH_{ar}$; 52.5 & 49.8 $CH_2$; 13.2 & 10.6 $CH_3$
[1] bei Angabe der NMR-Verschiebungen wird generell die Verschiebung des zum Hauptisomer gehörenden Peaks zuerst aufgeführt.

**N-(4-Chlor-3(5)-methyl-pyrazol-1-ylmethyl)-formamid 2**

$R_F$: 0,25 (Ethylacetat)
$^1$H-NMR ($CDCl_3$/300 MHz) δ /ppm: 8.23 & 8.20 (s, 1H, CH=O); 7.59 & 7.40 (s, 1H, $CH_{ar-om.}$); 5.40 & 5.47 (d, 2H, J=6.7 Hz, $CH_2$); 2.22 & 2.40 (s, 3H, $CH_3$)
$^{13}$C-NMR ($CDCl_3$/125 MHz) δ /ppm: 162.1 &161.4 C=O;, 147.2 & 136.9 (3(5)-$\underline{C}_{q,arom}$-$CH_3$); 138.3 & 129.6 ($CH_{arom}$); 110.7 & 109.9 ($\underline{C}_{q,arom}$-d); 54.4 & 52.2 $CH_2$; 12.3 & 10.2 $CH_3$

**N-(3(5),4-Dimethyl-pyrazol-1-ylmethyl)-formamid 3**

$R_F$: 0,14 (Ethylacetat)
$^1$H-NMR ($CDCl_3$/300 MHz) δ /ppm: 8.21 & 8.19 (s, 1H, CH=O); 7.35 & 7.25 (s, 1H, $CH_{ar-om.}$); 5.36 & 5.44 (d, 2H, J=6.5 Hz, $CH_2$); 2.14 & 2.32 (s, 3H, 3(5)-$CH_3$); 1.96 (s, 3H, 4-$CH_3$)
$^{13}$C-NMR ($CDCl_3$/125 MHz) δ /ppm: 162.1 & 161.5 C=O; 148.7 & 137.0 (3(5)-$\underline{C}_{q,arom}$-$CH_3$); 140.3 & 130.6 ($CH_{arom.}$); 115.3 & 114.4 (4-$\underline{C}_{q,arom}$-$CH_3$); 53.5 & 51.3 $CH_2$; 12.7 4-$CH_3$; 10.3 & 9.5 $CH_3$

**Ermittlung der nitrifikationshemmenden Wirken**

Die Prüfung der erfindungsgemäßen N-(1 H-Pyrazolyl-methyl)-formamide erfolgte in den in Tabelle 2 ausgewiesenen Konzentrationen. Dabei wurde die nitrifikationshemmende Wirkung in der Weise ermittelt, dass 100 g lufttrockener Boden auf 50% der maximalen Wasserkapazität eingestellt wurden. Hierzu wurden jeweils 10 mg Harnstoff-N und die ausgewiesene Wirkstoffmenge unter möglichst homogener Verteilung zugesetzt. Danach werden die so präparierten Gefäße unter standardisierten Bedingungen bei 20°C im dunklen Klimaraum aufbewahrt. Aus der zeitabhängigen Bestimmung des Umsatzes von Ammonium-N zu Nitratstickstoff im Vergleich zur Variante ohne Inhibitor wird die prozentuale Hemmung zum Tag X ermittelt. Der nachfolgend verwendete K-Wert verkörpert einen von den biologischen Schwankungen der Bodenaktivität unabhängigen Wert, da er auf die Wirkung eines Standards, in diesem Fall 3-Methylpyrazol mit einer Inhibitorkonzentration von 0,2% N-bezogen, durch Quotientenbildung relativiert wird. Der Ermittlung des K-Wertes ist die Berechnung des $t_{50}$ Wertes vorangestellt. Dieser Wert drückt den Zeitpunkt in Tagen aus, an dem die Nitrifikationshemmung für eine einzelne Substanz auf 50% zurückgegangen ist. Daraus leitet sich der K-Wert wie folgt ab:

$$K = \frac{t_{50} - \text{Wert der Testsubstanz}}{t_{50} - \text{Wert Standard}}$$

Tabelle 2: Nitrifikationshemmende Wirkung ausgewählter Verbindungen

| Nr. der Verbindung | Wirkstoffkonzentration | K-Wert |
|---|---|---|
| **1** | 0,2 | 0,76 |
| | 0,3 | 0,97 |
| **2** | 0,2 | 1,01 |
| | 0,3 | 1,37 |
| **3** | 0,2 | 0,82 |
| | 0,3 | 1,09 |

**Patentansprüche**

1. N-(1 H-Pyrazolyl-methyl)-formamide der allgemeinen Formel I:

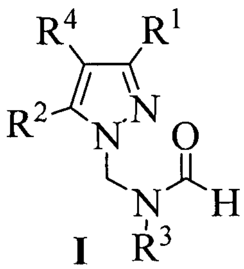

**I**

**dadurch gekennzeichnet, dass**

$R^4$ = H, Halogen, $C_1$-$C_4$-Alkyl oder $C_3$-$C_8$-Cycloalkyl ist,
$R^1$ und $R^2$ unabhängig voneinander für H, $C_1$-$C_4$-Alkyl oder $C_3$-$C_8$-Cycloalkyl steht,
$R^3$ für $C_1$-$C_4$-Alkyl, $C_3$-$C_8$-Cycloalkyl oder $C_6$-$C_{10}$-Aryl oder H steht.

2. N-(1H-Pyrazolyl-methyl)-formamide nach Anspruch 1, **dadurch gekennzeichnet, dass** unabhängig voneinander $R^1$ einen Methyl- und $R^4$ einen Methylrest darstellt und, vorzugsweise, $R^2$ H ist.

3. N-(1H-Pyrazolyl-methyl)-amide nach Anspruch 1, **dadurch gekennzeichnet, dass** $R^1$ einen Methylrest darstellt und $R^4$ für Chlor oder H steht und, vorzugsweise, $R^2$ H ist.

4. Verfahren zur Herstellung der N-(1H-Pyrazolyl-methyl)-formamide I nach den Ansprüchen 1 bis 3, **dadurch gekennzeichnet, dass** man die entsprechenden Pyrazole der allgemeinen Formel II

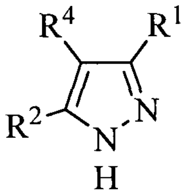

**II**

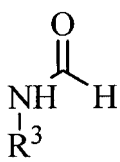

**IV**

wobei $R^1$, $R^2$ und $R^4$ oben genannte Bedeutung besitzen, mit Formaldehyd und Amiden der allgemeinen Formel IV, wobei $R^3$ die oben genannte Bedeutung besitzt, reagieren lässt.

5. Verfahren nach Anspruch 4, **dadurch gekennzeichnet, dass** man die Reaktion bei Temperaturen von 100 bis

200°C ohne Lösungsmittel in der Schmelze und mit einem Molverhältnis von Azol II zu Formaldehyd und Amid IV von 1:3:3 bis 1:1:1 durchführt.

6. Verfahren nach Anspruch 5, **dadurch gekennzeichnet, dass** man die Reaktion bei 125 bis 145°C durchführt.

7. Verfahren nach Anspruch 4, **dadurch gekennzeichnet, dass** man die Reaktion bei Temperaturen von 40 bis 110°C in einem organischen Lösungsmittel oder Wasser und mit einem Molverhältnis von Azol II zu Formaldehyd und Amid IV von 1:3:3 bis 1:1:1 durchführt.

8. Verfahren nach Anspruch 7, **dadurch gekennzeichnet, dass** man einen Lösungsvermittler aus der Gruppe der Alkohole oder Acetonitril zusetzt.

9. Verfahren zur Herstellung der N-(1 H-Pyrazolyl-methyl)-formamide I nach den Ansprüchen 1 bis 3, **dadurch gekennzeichnet, dass** man die entsprechenden 1-Hydroxymethylazole der allgemeinen Formel V

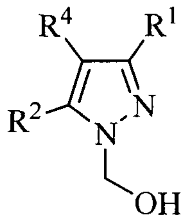

V

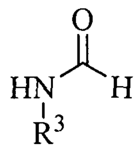

IV

wobei $R^1$, $R^2$, $R^4$ oben genannte Bedeutung besitzen, mit Amiden der allgemeinen Formel IV, wobei $R^3$ die oben genannte Bedeutung besitzt, reagieren lässt.

10. Verfahren nach Anspruch 9, **dadurch gekennzeichnet, dass** man die Reaktion unter Zusatz von 0,2 bis 10% einer geeigneten Säure, vorzugsweise eines saueren Ionenaustauschers, durchführt.

11. Verfahren nach Anspruch 9, **dadurch gekennzeichnet, dass** die Reaktion ohne Lösungsmittel durchgeführt wird.

12. Verwendung von N-(1H-Pyrazolyl-methyl)-formamiden entsprechend der allgemeinen Formel (I)

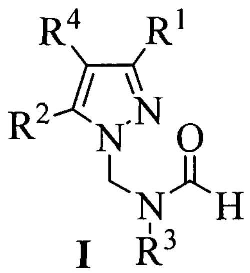

I

wobei $R^4$ = H, Halogen, $C_1$-$C_4$-Alkyl oder $C_3$-$C_8$-Cycloalkyl ist,
$R^1$ und $R^2$ unabhängig voneinander für H, $C_1$-$C_4$-Alkyl oder $C_3$-$C_8$-Cycloalkyl steht,
$R^3$ für $C_1$-$C_4$-Alkyl, $C_3$-$C_8$-Cycloalkyl oder $C_6$-$C_{10}$-Aryl oder H steht,

als Mittel zur Hemmung bzw. Steuerung der Nitrifikation.

13. Zusammensetzung, aufweisend die N-(1H-Pyrazolyl-methyl)-formamide nach den Ansprüchen 1, 2 und 3 sowie gegebenenfalls weitere Nitrifikationsinhibitoren und/oder Ureaseinhibitoren in Kombination mit festen und flüssigen ammonium- und/oder amidhaltigen Düngemitteln.

14. Zusammensetzung nach Anspruch 13, **dadurch gekennzeichnet, dass** die N-(1H-Pyrazolyl-methyl)-formamide

sowie die gegebenenfalls weiteren Nitrifikationsinhibitoren und/oder Ureaseinhibitoren in einer Gesamtaufwandmenge von 0,01 bis 10,0 Gewichtsprozent, berechnet auf den reduzierten Stickstoffanteil des amid- und/ oder ammoniumhaltigen Düngemittels, eingesetzt werden.

**15.** Zusammensetzung nach Anspruch 14, **dadurch gekennzeichnet, dass** die N-(1 H-Pyrazolyl-methyl)-formamide in einer Aufwandmenge von 0,01 bis 3,0 Gewichtsprozent eingesetzt werden.

**16.** Zusammensetzung nach den Ansprüchen 13 bis 15, **dadurch gekennzeichnet, dass** die N-(1H-Pyrazolyl-methyl)-formamide allein oder im Gemisch mit weiteren Zusatzstoffen in die Düngemittelschmelze oder -slurry während des Formgebungsprozesses eingebracht worden sind und auf diese Weise homogen im Dünger verteilt vorliegen.

**17.** Zusammensetzung nach den Ansprüchen 13 bis 15, **dadurch gekennzeichnet, dass** die N-(1H-Pyrazolyl-methyl)-formamide ggf. mit weiteren Zusatzstoffen in Form einer Lösung oder gemeinsam mit einem geeigneten Konditionierungsmittel auf die Oberfläche der Düngemittelgranulate aufgebracht worden sind und gegebenenfalls anschließend das Lösungsmittel durch Trocknung entfernt worden ist.

## Claims

**1.** N-(1H-pyrazolyl-methyl)-formamides of the general formula I:

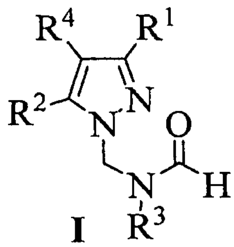

**I**

**characterised in that**

$R^4$ = H, halogen, $C_1$-$C_4$ alkyl or $C_3$-$C_8$-cycloalkyl,
$R^1$ and $R^2$ independently represent H, $C_1$-$C_4$ alkyl or $C_3$-$C_8$ cycloalkyl,
$R^3$ is $C_1$-$C_4$ alkyl, $C_3$-$C_8$ cycloalkyl or $C_6$-$C_{10}$ aryl or H.

**2.** N-(1H-pyrazolyl-methyl)-formamides of claim 1, **characterised in that** independently of one another $R^1$ represents a methyl and $R^4$ a methyl residue and, preferably, $R^2$ is H.

**3.** N-(1H-pyrazolyl-methyl)-amides of claim 1, **characterised in that** $R^1$ represents a methyl residue and $R^4$ is chlorine or H, and, preferably, $R^2$ is H.

**4.** The method for the production of N-(1H-pyrazolyl-methyl)-formamides I of claims 1 to 3, **characterised in that** the corresponding pyrazoles of the general formula II

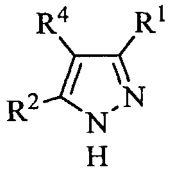

**II**

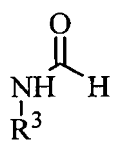

**IV**

wherein $R^1$, $R^2$ and $R^4$ are defined as above, react with formaldehyde and amides of the general formula IV, wherein

$R_3$ has the meaning given above.

5. The method of claim 4, **characterised in that** the reaction is carried out at temperatures of 100 to 200°C without solvent in the melt and with a molar ratio of azole II to formaldehyde and amide IV of 1:3:3 to 1:1:1.

6. The method of claim 5, **characterised in that** the reaction is carried out at 125 to 145°C.

7. The method of claim 4, **characterised in that** the reaction is carried out at temperatures of 40 to 110°C in an organic solvent or water and with a molar ratio of a zole II to formaldehyde and amide IV of 1:3:3 to 1:1:1.

8. The method of claim 7, **characterised in that** a solubilizer from the group of the alcohols or acetonitrile is added.

9. The method for the production of N-(1H-pyrazolyl-methyl)-formamides I of claims 1 to 3, **characterised in that** the respective 1-hydroxymethyl-azole of the general formula V

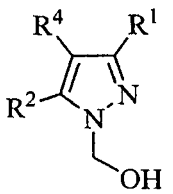

V

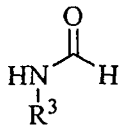

IV

wherein $R^1$, $R^2$ and $R^4$ are defined as above, react with amides of the general formula IV, wherein $R^3$ has the meaning given above.

10. The method of claim 9, **characterised in that** the reaction is carried out adding 0.2 to 10% of a suitable acid, preferably an acidic ion exchanger.

11. The method of claim 9, **characterised in that** the reaction is carried out without a solvent.

12. The use of N-(1H-pyrazolyl-methyl)-formamides corresponding to the general formula (I)

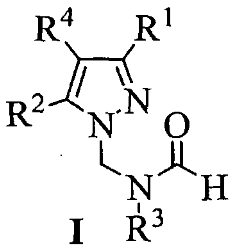

I

wherein $R^4$ = H, halogen, $C_1$-$C_4$ alkyl or $C_3$-$C_8$ cycloalkyl,
$R^1$ and $R^2$ independently represent H, $C_1$-$C_4$ alkyl or $C_3$-$C_8$ cycloalkyl,
$R^3$ is $C_1$-$C_4$ alkyl, $C_3$-$C_8$ cycloalkyl or $C_6$-$C_{10}$ aryl or H,

as a means to inhibit or control nitrification.

13. The composition comprising the N-(1H-pyrazolyl-methyl)-formamides of claims 1, 2 and 3 and optionally further nitrification inhibitors and/or urease inhibitors in combination with solid and liquid ammonium-containing and/or amide-containing fertilizers.

**14.** The composition of claim 13, **characterised in that** the N-(1H-pyrazolyl-methyl)-formamides and optionally further nitrification inhibitors and/or urease inhibitors are used at a total application rate of 0.01 to 10.0 percent by weight, calculated to the reduced nitrogen portion of the amide-containing and/or ammonium-containing fertilizer.

**15.** The composition of claim 14, **characterised in that** the N-(1H-pyrazolyl-methyl)-formamides are used at an application rate of 0.01 to 3.0 percent by weight.

**16.** The composition of claims 13 to 15, **characterised in that** the N-(1H-pyrazolylmethyl)-formamides alone or in a mixture with further additives were introduced into the fertilizer melt or slurry during the molding process and are in this manner homogeneously mixed with the fertilizer.

**17.** The composition of claims 13 to 15, **characterised in that** the N-(1H-pyrazolylmethyl)-formamides, optionally with further additives in the form of a solution or together with a suitable conditioning agent, were applied to the surface of the fertilizer granules and that optionally the solvent is then removed by drying.

## Revendications

**1.** Formamide N-(1 H-Pyrazolyl-methyl) de formule générale 1 :

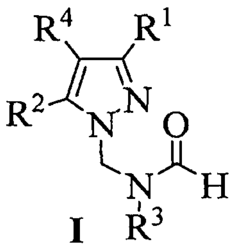

I

**caractérisée par le fait que**

$R^4$ = H, halogène, Ci-C4-Alkyle ou $C_3$-$C_8$-Cycloalkyle,
$R^1$ et $R^2$ représentent, indépendamment l'un de l'autre, H, $C_1$-$C_4$-Alkyle ou $C_3$-$C_8$ Cycloalkyle,
$R^3$ représente $C_1$-$C_4$-Alkyle, $C_3$-$C_8$-Cycloalkyle ou $C_6$-$C_{10}$-Aryle ou H.

**2.** Formamide N-(1H-Pyrazolyl-methyl) selon la revendication 1, **caractérisée par le fait que**, indépendamment l'un de l'autre, $R^1$ représente un méthyle et $R^4$ un reste de méthyle et, de préférence, $R^2$ est H.

**3.** Amide N-(1H-Pyrazolyl-methyl) selon la revendication 1, **caractérisée par le fait que** $R^1$ représente un reste de méthyle et $R^4$ représente le chlore ou H et, de préférence, $R^2$ est H.

**4.** Procédé de production de la formadide N-(1H-Pyrazolyl-methyl) I selon les revendications 1 à 3, **caractérisé par le fait qu'**on laisse réagir la pyrazole correspondante de la formulation générale II

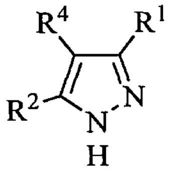

II

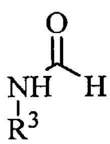

IV

où $R^1$ , $R^2$ et $R^4$ répondent à la définition donnée plus haut, avec le formaldéhyde et les amides de la formulation générale IV, où $R_3$ répond à la définition donnée plus haut.

**5.** Procédé selon la revendication 4, **caractérisé par le fait que** l'on procède à la réaction à des températures de 100 à 200°C sans solvant dans la fonte et avec un rapport entre Azol II et formaldéhyde et amide IV de 1:3:3 à 1:1:1.

**6.** Procédé selon la revendication 5, **caractérisé par le fait que** l'on procède à la réaction à des températures de 125 à 145°C.

**7.** Procédé selon la revendication 4, **caractérisé par le fait que** l'on procède à la réaction à des températures de 40 à 110°C dans un solvant organique ou de l'eau et avec un rapport entre Azol II et formaldéhyde et amide IV de 1:3:3 à 1:1:1.

**8.** Procédé selon la revendication 7, **caractérisé par le fait que** l'on ajoute un solvant du groupe des alcools ou acétonitrile.

**9.** Procédé de production de la formamide N-(1H-Pyrazolyl-methyl) I selon les revendications 1 à 3, **caractérisé par le fait que** l'on laisse réagir l'hydroxyméthylazole 1 correspondante de la formulation générale V

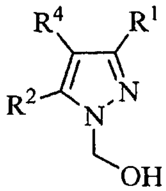

**V**

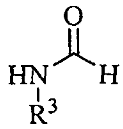

**IV**

où $R^1$, $R^2$, $R^4$ répondent à la définition donnée plus haut, avec les amides de la formulation générale IV, où $R^3$ répond à la définition donnée plus haut.

**10.** Procédé selon la revendication 9, **caractérisé par le fait que** l'on procède à la réaction en ajoutant 0,2 à 10 % d'un acide adapté, de préférence un échangeur d'ions acidique.

**11.** Procédé selon la revendication 9, **caractérisé par le fait que** la réaction est effectuée sans solvant.

**12.** Utilisation de formamide N-(1H-Pyrazolyl-methyl) de formule générale I

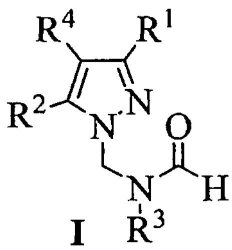

**I**

où $R^4$ = H, halogène, $C_1$-$C_4$-Alkyle ou $C_3$-$C_8$-Cycloalkyle,
$R^1$ et $R^2$ représentent indépendamment l'un de l'autre H, $C_i$-$C_4$-Alkyle ou $C_3$-$C_8$-Cycloalkyle,
$R^3$ représente $C_1$-$C_4$-Alkyle, $C_3$-$C_8$-Cycloalkyle ou $C_6$-$C_1$0-Aryle
ou H, comme moyen d'empêcher ou de piloter la nitrification.

**13.** Composé présentant la formamide N-(1H-Pyrazolyl-methyl) selon les revendications 1, 2 et 3 ainsi, le cas échéant, que d'autres inhibiteurs de nitrification et/ou des inhibiteurs d'urée en combinaison avec des engrais solides et liquides contenant de l'ammonium et/ou des amides.

**14.** Composé selon la revendication 13, **caractérisé par le fait que** la formamide N-(1H-Pyrazolyl-methyl) et, le cas échéant, les autres inhibiteurs de nitrification et/ou les inhibiteurs d'urée sont utilisés selon une quantité totale de

0,01 à 10,0 pour cent du poids, calculé sur la partie d'azote réduite de l'engrais contenant de l'ammonium et/ou des amides.

**15.** Composé selon la revendication 14, **caractérisé par le fait que** la formamide N-(1 H-Pyrazolyl-methyl) est utilisée selon une quantité de 0,01 à 3,0 pour cent du poids.

**16.** Composé selon les revendications 13 à 15, **caractérisé par le fait que** la formamide N-(1H-Pyrazolyl-methyl) est ajoutée seule ou mélangée à d'autres substances dans la boue d'engrais pendant le formage et est ainsi répartie de manière homogène dans l'engrais.

**17.** Composé selon les revendications 13 à 15, **caractérisé par le fait que** la formamide N-(1H-Pyrazolyl-methyl) est le cas échéant ajoutée avec d'autres substances sous forme de solution ou en combinaison avec un conditionneur adapté sur la surface des granulés de l'engrais et que, le cas échéant, la solution est éliminée par séchage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3635690 A
- US 4969946 A
- US 4523940 A
- EP 166420 A
- JP OS7104135 B
- US 3697244 A
- DE OS2745833 A
- EP 508191 A
- DD 292232
- DE OS4018395 A
- EP 160804 A
- DE OS3704359 A
- SU 1470737
- DE OS4446194 A
- EP 808297 A
- EP 529473 A
- WO 9805607 A
- DE 3704359 A
- SU 1470737 A
- DE OS10117821 A1
- DE OS10135423 A1
- DE 10343277 A1
- DE 19822340 A1
- WO OS2835158 A
- WO OS19913475 A


### In der Beschreibung aufgeführte Nicht-Patentliteratur


- *J. Hüttel Chem. Ber.,* 1952, vol. 85, 820
- **L. MAIER.** *Phosphorus Sulfur,* 1987, vol. 33, 41